# EUROPÄISCHE PATENTSCHRIFT

(11) **EP 4 196 081 B1**
(45) Veröffentlichungstag und Bekanntmachung des Hinweises auf die Patenterteilung: **30.09.2026**
(21) Anmeldenummer: 21745259.8
(22) Anmeldetag: 07.07.2021
(51) Int. Cl.: A61K 8/06, A61K 8/34, A61K 8/37, A61K 8/55, A61Q 19/00

(54) **KOSMETISCHE ETHANOL-IN-GLYCERIN-EMULSION**
COSMETIC ETHANOL-IN-GLYCEROL EMULSION
ÉMULSION COSMÉTIQUE D'ÉTHANOL-DANS-GLYCÉROL

(30) Priorität: 13.08.2020 DE 102020210307
(43) Veröffentlichungstag der Anmeldung: 21.06.2023
(73) Patentinhaber: Beiersdorf AG, 22529 Hamburg (DE)
(72) Erfinder: SIVACILAR, Berkay Daniel, 20539 Hamburg (DE); GOULET-HANSSENS, Alexis, 20255 Hamburg (DE); VON WEDEL-PARLOW, Magdalena, 22527 Hamburg (DE)
(74) Vertreter: Beiersdorf AG
(86) Internationale Anmeldenummer: PCT/EP2021/068801
(87) Internationale Veröffentlichungsnummer: WO 2022/033773

(56) Entgegenhaltungen:
- EP-A1- 2 515 828
- FR-A1- 3 038 514
- US-A- 5 020 530
- US-B2- 9 168 283

## Beschreibung

Die vorliegende Erfindung betrifft eine kosmetische Emulsion aus Ethanol, einer Glycerinphase, einem Emulgator und gegebenenfalls weiteren kosmetischen Wirk-Hilfs und Zusatzstoffen, welche im Ethanol oder der Glycerinphase gelöst vorliegen sowie die Verwendung derartiger Emulsionen als kosmetische Zubereitung.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Hautpflegeprodukte (sowie einige Hautreinigungsprodukte) bestehen in der Regel aus Emulsionen. Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Neben Emulsionen, die aus einer Wasserphase und einer Ölphase gebildet werden, sind grundsätzlich auch andere Systeme von mit Ölen nicht mischbaren flüssigen Phasen denkbar, die in der Kosmetik bisher jedoch -nicht zuletzt wegen formulierungstechnischer Schwierigkeitenkeine Rolle spielen.

Es war daher die Aufgabe der vorliegenden Erfindung, ein alternatives Emulsionssystem für die Kosmetik zu entwickeln, dass Lager- und Temperatur-stabil ist und eine angenehme Sensorik aufweist. Insbesondere Phasentrennungen im Verlaufe der Lagerung sollten vermieden werden. Darüber hinaus sollten sich kosmetische Wirkstoffe, wie beispielsweise UV-Filter, Antioxidantien, Haut-befeuchtende Stoffe, Antifaltenwirkstoffe, Parfümstoffe etc. stabil und in wirksamer Menge einarbeiten lassen.

Nachteilig an kosmetischen Emulsionen des Standes der Technik ist darüber hinaus der Umstand, dass der Gehalt an Wasser dazu führt, dass die Zubereitung nicht mehr mikrobiell stabil ist und das Kosmetikum zur Haltbarmachung mit Konservierungsmitteln versetzt werden muss. An sich ist der Einsatz von Konservierungsmitteln in Kosmetika unkritisch, doch wird von unterschiedlicher Seite immer wieder deren Verträglichkeit in Frage gestellt. Ob und in welchem Umfang derlei Bedenken einer wissenschaftlichen Überprüfung Stand halten, kann im Rahmen der vorliegenden Offenbarung dahingestellt bleiben. Tatsache ist jedoch, dass ein nicht unbedeutender Teil der Verbraucher "Konservierungsmittel-freie" Produkte wünscht.

Es war daher die Aufgabe der vorliegenden Erfindung, eine kosmetische Emulsion zu entwickeln, die frei ist von Konservierungsstoffen. Dabei werden erfindungsgemäß unter Konservierungsstoffen diejenigen Verbindungen verstanden, die zum Prioritätszeitpunkt dieser Anmeldung in der Positiv-Liste der Kosmetikrichtlinie der Europäischen Union aufgelistet in Anhang VI aufgelistet sind.

Aufgrund ihres guten Emulgier-Vermögens stellen die Polyethylenglycol- und Polypropylenglycol-Ether und -Ester eine viel verwendete Gruppe von kosmetischen Emulgatoren dar. Nachteilig am Stande der Technik ist jedoch der Umstand, dass diese Emulgatoren im Verdacht stehen, die Hautpenetration von Stoffen begünstigen, die nicht in die tieferen Hautschichten eindringen sollen. Ob und in welchem Umfang diese Eigenschaft ein tatsächliches Problem für die Anwender darstellt, kann im Rahmen der vorliegenden Offenbarung ebenfalls dahingestellt bleiben. Tatsache ist auch hier, dass der Einsatz von Polyethylenglycol bzw. Polypropylenglycol -ethern und - estern bei den Verbrauchern zunehmend unerwünscht ist und es die Aufgabe der vorliegenden Erfindung war, auf diese weit verbreiteten Emulgator-Systeme zu verzichten.

Üblicherweise werden die wässrigen Phasen einer kosmetischen Emulsion mit Trinkwasser hergestellt. Aufgrund der unterschiedlichen Gehalte an Alkali- und Erdalkalisalzen kommt es bei der Herstellung der wässrigen Phasen immer wieder zu Problemen, da die Salzgehalte die Stabilität der Emulsion beeinflussen. Um an unterschiedlichen Herstellungsorten (mit unterschiedlichen Wasserqualitäten) die gleiche kosmetische Rezeptur herstellen zu können, muss das eingesetzte Trinkwasser in der Regel erst einmal einem aufwendigen Aufbereitungsprozess (z. B. Ionenaustausch) unterworfen werden, um vergleichbare Wasserqualitäten zu erzeugen mit denen dann die Emulsion hergestellt werden kann.

Es war daher die Aufgabe der vorliegenden Erfindung, eine kosmetische Emulsion zu entwickeln, die ohne den Einsatz von Trinkwasser in der wässrigen Phase auskommt.

Überraschend gelöst werden die Aufgaben durch eine kosmetische Ethanol-in-Glycerin-Emulsion gemäß Anspruch 1.

Überraschend gelöst werden die Aufgaben ferner durch die Verwendung einer Ethanol-in-Glycerin-Emulsion gemäß Anspruch 2.

Überraschend gelöst werden die Aufgaben nicht zuletzt durch ein Verfahren gemäß Anspruch 3.

Zwar kennt der Stand der Technik die US 9,168,283 B2, FR 3 038 514 A1, EP 2 515 828 A1 und US 5 020 530 A, doch konnten diese Schriften nicht den Weg zur vorliegenden Erfindung weisen.

Im Rahmen der vorliegenden Offenbarung beziehen sich die Formulierungen "erfindungsgemäß", "erfindungsgemäße Zubereitung" etc. immer auf die erfindungsgemäßen Zubereitungen, Verfahren und Verwendungen, d.h. auch auf Zubereitungen, in denen die erfindungsgemäßen Verwendungen verwirklicht werden, sowie Zubereitungen, mit denen das erfindungsgemäße Verfahren verwirklicht wird.

Es ist erfindungsgemäß vorteilhaft, wenn der Ethanolgehalt der Zubereitung 5 bis 25 Gewichts-%, bezogen auf das Gesamtgewicht der Emulsion, beträgt. Dabei ist ein Ethanolgehalt von 8 bis 12 Gewichts-%, bezogen auf das Gesamtgewicht der Zubereitung, erfindungsgemäß bevorzugt. Besonders gute Ergebnisse wurden mit einem Ethanolgehalt von 10 Gewichts-%, bezogen auf das Gesamtgewicht der Zubereitung, erzielt.

Es ist vorteilhaft im Sinne der vorliegenden Erfindung, wenn die Glycerinphase ein oder mehrere Öle gewählt aus der Gruppe der Verbindungen und Ethylhexyl-2-cyano-3,3-di-phenylacrylat (INCI: Octocrylen), 2-Ethylhexyl 2-hydroxybenzoat (INCI: Ethylhexyl Salicylate) und 3,3,5-Trimethylcyclohexyl 2-hydroxybenzoat (INCI: Homosalate) enthält. Es war dabei für den Fachmann keinesfalls naheliegend, dass die Einarbeitung dieser UV-Filter überhaupt möglich ist.

Es ist erfindungsgemäß vorteilhaft, wenn die Zubereitung Ethylhexyl-2-cyano-3,3-diphenyl-acrylat (INCI: Octocrylen) in einer Konzentration von 5,0 bis 25,0 Gewichts-%, bezogen auf das Gesamtgewicht der Zubereitung, enthält.

Es ist erfindungsgemäß vorteilhaft, wenn die Zubereitung 3,3,5-Trimethylcyclohexyl 2-hydroxybenzoat (INCI: Homosalate) in einer Konzentration von 1,0 bis 7,5 Gewichts-%, bezogen auf das Gesamtgewicht der Zubereitung, enthält.

Es ist erfindungsgemäß vorteilhaft, wenn die Zubereitung 2-Ethylhexyl 2-hydroxybenzoat (INCI: Ethylhexyl Salicylate) in einer Konzentration von 1,0 bis 7,5 Gewichts-%, bezogen auf das Gesamtgewicht der Zubereitung, enthält.

Insbesondere ist es erfindungsgemäß bevorzugt, wenn die Glycerinphase allein 2-Ethylhexyl 2-hydroxybenzoat (INCI: Ethylhexyl Salicylate) in einer Konzentration von 1,0 bis 7,5 Gewichts-%, bezogen auf das Gesamtgewicht der Zubereitung, enthält.

Im Hinblick auf den Einsatz von Ethylhexyl-2-cyano-3,3-diphenylacrylat (INCI: Octocrylen) ist festzuhalten, dass erfindungsgemäß 2 Alternativen denkbar sind: Ein Gehalt von Octocrylen und der Verzicht auf Octocrylen. Erfindungsgemäß bevorzugt ist dabei die Variante ohne Octocrylen.

Es ist erfindungsgemäß vorteilhaft, wenn die Zubereitung Glycerin in einer Konzentration von 50 bis 95 Gewichts-%, bezogen auf das Gesamtgewicht der Zubereitung, enthält.

Es ist erfindungsgemäß bevorzugt, wenn die Zubereitung Glycerin in einer Konzentration von 60 bis 90 Gewichts-%, bezogen auf das Gesamtgewicht der Zubereitung, enthält.

Die erfindungsgemäße Emulsion kann darüber hinaus UV-Filter enthalten. Diese können beispielsweise gewählt werden aus der Gruppe der Verbindungen: 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-Benzylidencampher; 4-(Dimethylamino)-benzoesäure(2-ethylhexyl)ester; 4-(Dimethylamino)benzoesäureamylester; 4-Methoxybenzalmalonsäuredi(2-ethylhexyl)ester; 4- 4-Methoxyzimtsäureisoamylester; 2-Hydroxy-4-methoxy-4'-methylbenzophenon; 2,2'-Dihydroxy-4-methoxybenzophenon; 4-(tert.-Butyl)-4'-methoxydibenzoylmethan; Homomenthylsalicylat; 2-Ethylhexyl-2-hydroxybenzoat; Dimethicodiethylbenzalmalonat; 3-(4-(2,2-bis Ethoxycarbonylvinyl)-phe-noxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 2,4,6-Tris-(biphenyl)-1,3,5-triazin; 2,4-Bis-(4'- Di-neopentylaminobenzalmalonat)-6-(4"-butylaminobenzoat)-s-triazin, Titandioxid, Zinkoxid und den Merocyaninen.

Erfindungsgemäß bevorzugt ist es dabei, wenn die Zubereitung einen oder mehrere UV-Filter gewählt aus der Gruppe der Verbindungen 4-(tert.-Butyl)-4'-methoxydibenzoylmethan, Hexyl 2-[4-(diethylamino)-2-hydroxybenzoyl]benzoate (INCI: Diethylamino hydroxybenzoyl hexyl benzoate), 2,4,6-Tris-[anilino-(p-carbo-2'-ethyl-1'-hexyloxy)]-1,3,5-triazin (INCI: Ethylhexyl Triazone), 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol methoxyphenyl Triazine),enthält.

Die erfindungsgemäße Emulsion kennt insbesondere zwei erfindungsgemäß vorteilhafte Emulgator-Systeme: Zum einen können Polyglycerylester eingesetzt werden, zum anderen Sojalecithine.

Werden Polyglycerylester als Emulgator eingesetzt, so sind diese erfindungsgemäß vorteilhaft dadurch gekennzeichnet, dass als Emulgator ein Polyglyceryl-10 Fettsäureester eingesetzt wird.

Dabei ist es erfindungsgemäß bevorzugt, wenn Polyglyceryl-10 Fettsäureester in einer Konzentration von 0,1 bis 1,5 Gewichts-%, bezogen auf das Gesamtgewicht der Zubereitung, eingesetzt werden.

Als erfindungsgemäß bevorzugte Polyglyceryl-10 Fettsäureester werden dabei Polyglyceryl-10 Stearat (INCI Polyglyceryl-10 Stearate) und/oder Polyglyceryl-10 Oleat (INCI: Polyglyceryl-10 Oleate) eingesetzt.

Erfindungsgemäß besonders bevorzugt ist dabei der Einsatz von oder Polyglyceryl-10 Oleat (INCI: Polyglyceryl-10 Oleate).

Werden hingegen Sojalecithine als Emulgatoren eingesetzt, so ist es erfindungsgemäß vorteilhaft, wenn Sojalecithin in einer Konzentration von 0,1 bis 1,5 Gewichts-%, bezogen auf das Gesamtgewicht der Zubereitung, eingesetzt wird.

Erfindungsgemäß bevorzugt ist es, wenn die Zubereitung frei ist von 3-(4-Methylbenzyliden)-campher, 2-Hydroxy-4-methoxybenzophenon (INCI: Oxybenzon), 4-Methoxyzimtsäure-2-ethylhexylester (INCI Octylmethoxycinnamate), 2-Ethylhexyl-2-cyano-3,3-diphenylacrylat, Homomenthylsalicylat, Parabenen (insbesondere Propyl- und Butylparaben), Methylisothiazolinon, Chlormethylisothiazolinon und DMDM-Hydantoin, Polyethylenglycolethern oder Polyethylenglycolestern, sowie frei ist von Mineralölen, Silikonölen und Mineralwachsen.

Erfindungsgemäß vorteilhafte Ausführungsformen der vorliegenden Erfindung sind nicht zuletzt dadurch gekennzeichnet, dass die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Panthenol, Magnolol, Honokiol, Tocopherol, Tocopherylacetat, Dihydroxyaceton; Polydocanol, Thiamidol, Glycerylglycose, Hyaluronsäure und/oder deren Salze und/oder Licochalcon A enthält.

**Im** Hinblick auf die Viskosität ist es erfindungsgemäß von Vorteil, wenn die erfindungsgemäße Emulsion eine Viskosität von weniger als 1500 mPas (gemessen mit *Rheomat R123* von "proRheo" bei 25°C, bei konstanter Drehzahl für 30 Sekunden) aufweist.

Das erfindungsgemäße Verfahren ist erfindungsgemäß vorteilhaft dadurch gekennzeichnet, dass die Homogenisierung mit Hilfe eines Ultra-Turrax durchgeführt wird. Hierfür kommt beispielsweise das Model T-25 in Frage. Üblicherweise erfolgt diese Homogenisierung dann bei 11000 U/min. während einer Dauer von etwa 5 Minuten.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| INCI | Beispiel 1 | Beispiel 2 | Beispiel 3 | Beispiel 4 | Beispiel 5 | Beispiel 6 |
|---|---|---|---|---|---|---|
| Alcohol Denat. + Aqua | 10,00 | 9,82 | 10,06 | 10,00 | 10,00 | 10,00 |
| Polyglyceryl-10 Oleate | 1,00 | 0,98 | 0,93 | 1,00 | 1,00 | 1,00 |
| Glycerin | 89,00 | 72,83 | 65,79 | 84,00 | 84,00 | 84,00 |
| Butyl methoxydibenzoylmethane | | 4,09 | 3,87 | | | |
| Octocrylene | | 12,28 | 19,35 | | | |
| Bis Ethylhexyloxyphenol Methoxyphenyl Triazine | | | | 5,00 | | |
| Ethylhexyl Triazone | | | | | 5,00 | |
| Homosalate | | | | | | 5,00 |

## Patentansprüche

1. Kosmetische Ethanol-in-Glycerin-Emulsion aus
a) Ethanol
b) einer Glycerinphase
c) einem Emulgator
d) gegebenenfalls weiteren kosmetischen Wirk-Hilfs und Zusatzstoffen, welche im Ethanol oder der Glycerinphase gelöst vorliegen, **dadurch gekennzeichnet, dass** der Wassergehalt der Zubereitung weniger als 5 Gewichts-%, bezogen auf das Gesamtgewicht der Emulsion, beträgt, sowie **dadurch gekennzeichnet, dass** als Emulgator ein oder mehrere Polyglycerylester oder Sojalecithin eingesetzt werden.

2. Verwendung einer Ethanol-in-Glycerin-Emulsion aus
a) Ethanol
b) einer Glycerinphase
c) einem Emulgator, **dadurch gekennzeichnet, dass** als Emulgator ein oder mehrere Polyglycerylester oder Sojalecithin eingesetzt werden,
d) gegebenenfalls weiteren kosmetischen Wirk-Hilfs und Zusatzstoffen, welche im Ethanol oder der Glycerinphase gelöst vorliegen, als Kosmetikum, **dadurch gekennzeichnet, dass** der Wassergehalt der Zubereitung weniger als 5 Gewichts-%, bezogen auf das Gesamtgewicht der Emulsion, beträgt.

3. Verfahren zur Herstellung einer Ethanol-in-Glycerin-Emulsion, **dadurch gekennzeichnet, dass** in einem ersten Schritt der Emulgator im Ethanol gelöst wird, in einem zweiten Schritt die Glycerinphase zur Ethanol/Emulgatormischung unter Rühren zugesetzt wird und anschließend homogenisiert wird, **dadurch gekennzeichnet, dass** der Wassergehalt der Zubereitung weniger als 5 Gewichts-%, bezogen auf das Gesamtgewicht der Emulsion, beträgt, sowie **dadurch gekennzeichnet, dass** als Emulgator ein oder mehrere Polyglycerylester oder Sojalecithin eingesetzt werden.

4. Emulsion nach Anspruch 1, Verwendung nach Anspruch 2 oder Verfahren nach Anspruch 3, **dadurch gekennzeichnet, dass** der Ethanolgehalt der Zubereitung 5 bis 25 Gewichts-%, bezogen auf das Gesamtgewicht der Emulsion, beträgt.

5. Emulsion, Verwendung oder Verfahren nach einem der vorhergehenden Ansprüche, **dadurch gekennzeichnet, dass** die Glycerinphase ein oder mehrere Öle gewählt aus der Gruppe der Verbindungen und Ethylhexyl-2-cyano-3,3-diphenylacrylat (INCI: Octocrylen), 2-Ethylhexyl 2-hydroxybenzoat (INCI: Ethylhexyl Salicylate) und 3,3,5-Trimethylcyclohexyl 2-hydroxybenzoat (INCI: Homosalate) enthält.

6. Emulsion, Verwendung oder Verfahren nach einem der vorhergehenden Ansprüche, **dadurch gekennzeichnet, dass** die Glycerinphase 2-Ethylhexyl 2-hydroxybenzoat (INCI: Ethylhexyl Salicylate) in einer Konzentration von 1,0 bis 7,5 Gewichts-%, bezogen auf das Gesamtgewicht der Zubereitung, enthält.

7. Emulsion, Verwendung oder Verfahren nach einem der vorhergehenden Ansprüche, **dadurch gekennzeichnet, dass** die Zubereitung Glycerin in einer Konzentration von 60 bis 90 Gewichts-%, bezogen auf das Gesamtgewicht der Zubereitung, enthält.

8. Emulsion, Verwendung oder Verfahren nach einem der vorhergehenden Ansprüche, **dadurch gekennzeichnet, dass** die Zubereitung einen oder mehrere UV-Filter gewählt aus der Gruppe der Verbindungen 4-(tert.-Butyl)-4'-methoxydibenzoylmethan, Hexyl 2-[4-(diethylamino)-2-hydroxybenzoyl]benzoate (INCI: Diethylamino hydroxybenzoyl hexyl benzoate), 2,4,6-Tris-[anilino-(p-carbo-2'-ethyl-1'-hexyloxy)]-1,3,5-triazin (INCI: Ethylhexyl Triazone), 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine),enthält.

9. Emulsion, Verwendung oder Verfahren nach einem der vorhergehenden Ansprüche, **dadurch gekennzeichnet, dass** als Emulgator ein Polyglyceryl-10 Fettsäureester eingesetzt wird.

10. Emulsion, Verwendung oder Verfahren nach einem der vorhergehenden Ansprüche, **dadurch gekennzeichnet, dass** als Emulgator ein Polyglyceryl-10 Fettsäureester in einer Konzentration von 0.1 bis 1,0 Gewichts-%, bezogen auf das Gesamtgewicht der Zubereitung, eingesetzt wird.

11. Emulsion, Verwendung oder Verfahren nach einem der vorhergehenden Ansprüche, **dadurch gekennzeichnet, dass** als Emulgator Polyglyceryl-10 Stearat (INCI Polyglyceryl-10 Stearate) und/oder Polyglyceryl-10 Oleat (INCI: Polyglyceryl-10 Oleate) eingesetzt werden.

12. Emulsion, Verwendung oder Verfahren nach einem der vorhergehenden Ansprüche, **dadurch gekennzeichnet, dass** als Emulgator Sojalecithin in einer Konzentration von 0.1 bis 1,0 Gewichts-%, bezogen auf das Gesamtgewicht der Zubereitung, eingesetzt wird.

13. Emulsion, Verwendung oder Verfahren nach einem der vorhergehenden Ansprüche, **dadurch gekennzeichnet, dass** die Zubereitung frei ist von 3-(4-Methylbenzyliden)-campher, 2-Hydroxy-4-methoxybenzophenon (INCI: Oxybenzon), 4-Methoxyzimtsäure-2-ethylhexylester (INCI Octylmethoxycinnamate), 2-Ethylhexyl-2-cyano-3,3-diphenylacrylat, Homomenthylsalicylat, Parabenen (insbesondere Propyl- und Butylparaben), Methylisothiazolinon, Chlormethyl-isothiazolinon und DMDM-Hydantoin, Polyethylenglycolethern oder Polyethylenglycolestern, sowie frei ist von Mineralölen, Silikonölen und Mineralswachsen.

14. Emulsion, Verwendung oder Verfahren nach einem der vorhergehenden Ansprüche, **dadurch gekennzeichnet, dass** die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Panthenol, Magnolol, Honokiol, Tocopherol, Tocopherylacetat, Dihydroxyaceton; Polydocanol, Thiamidol, Glycerylglycose, Hyaluronsäure und/oder deren Salze und/oder Licochalcon A enthält.

## Claims

1. Cosmetic ethanol-in-glycerin emulsion consisting of
a) ethanol
b) a glycerin phase
c) an emulsifier
d) optionally further cosmetic active ingredients, auxiliaries and additives which are dissolved in the ethanol or the glycerin phase, **characterized in that** the water content of the preparation is less than 5% by weight, based on the total weight of the emulsion, and **characterized in that** one or more polyglyceryl esters or soya lecithin are used as emulsifier.

2. Use of an ethanol-in-glycerin emulsion consisting of
a) ethanol
b) a glycerin phase
c) an emulsifier, **characterized in that** one or more polyglyceryl esters or soya lecithin are used as emulsifier,
d) optionally further cosmetic active ingredients, auxiliaries and additives which are dissolved in the ethanol or the glycerin phase, as a cosmetic, **characterized in that** the water content of the preparation is less than 5% by weight, based on the total weight of the emulsion.

3. Process for producing an ethanol-in-glycerin emulsion, **characterized in that** the emulsifier is dissolved in the ethanol in a first step, the glycerin phase is added to the ethanol/emulsifier mixture with stirring in a second step and then homogenization is performed, **characterized in that** the water content of the preparation is less than 5% by weight, based on the total weight of the emulsion, and **characterized in that** one or more polyglyceryl esters or soya lecithin are used as emulsifier.

4. Emulsion according to Claim 1, use according to Claim 2 or process according to Claim 3, **characterized in that** the ethanol content of the preparation is 5% to 25% by weight, based on the total weight of the emulsion.

5. Emulsion, use or process according to any of the preceding claims, **characterized in that** the glycerin phase comprises one or more oils selected from the group of compounds and ethylhexyl 2-cyano-3,3-diphenylacrylate (INCI: Octocrylene), 2-ethylhexyl 2-hydroxybenzoate (INCI: Ethylhexyl Salicylate) and 3,3,5-trimethylcyclohexyl 2-hydroxybenzoate (INCI: Homosalate).

6. Emulsion, use or process according to any of the preceding claims, **characterized in that** the glcyerin phase comprises 2-ethylhexyl 2-hydroxybenzoate (INCI: Ethylhexyl Salicylate) at a concentration of 1.0% to 7.5% by weight, based on the total weight of the preparation.

7. Emulsion, use or process according to any of the preceding claims, **characterized in that** the preparation comprises glycerin at a concentration of 60% to 90% by weight, based on the total weight of the preparation.

8. Emulsion, use or process according to any of the preceding claims, **characterized in that** the preparation comprises one or more UV filters selected from the group of compounds 4-(tert-butyl)-4'-methoxydibenzoylmethane, hexyl 2-[4-(diethylamino)-2-hydroxybenzoyl]benzoate (INCI: Diethylamino Hydroxybenzoyl Hexyl Benzoate), 2,4,6-tris[anilino-(p-carbo-2'-ethyl-1'-hexyloxy)]-1,3,5-triazine (INCI: Ethylhexyl Triazone), 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine).

9. Emulsion, use or process according to any of the preceding claims, **characterized in that** a polyglyceryl-10 fatty acid ester is used as emulsifier.

10. Emulsion, use or process according to any of the preceding claims, **characterized in that** a polyglyceryl-10 fatty acid ester is used as emulsifier at a concentration of 0.1% to 1.0% by weight, based on the total weight of the preparation.

11. Emulsion, use or process according to any of the preceding claims, **characterized in that** polyglyceryl-10 stearate (INCI: Polyglyceryl-10 Stearate) and/or polyglyceryl-10 oleate (INCI: Polyglyceryl-10 Oleate) are used as emulsifier.

12. Emulsion, use or process according to any of the preceding claims, **characterized in that** soya lecithin is used as emulsifier at a concentration of 0.1% to 1.0% by weight, based on the total weight of the preparation.

13. Emulsion, use or process according to any of the preceding claims, **characterized in that** the preparation is free of 3-(4-methylbenzylidene)camphor, 2-hydroxy-4-methoxybenzophenone (INCI: Oxybenzone), 2-ethylhexyl 4-methoxycinnamate (INCI: Octyl Methoxycinnamate), 2-ethylhexyl 2-cyano-3,3-diphenylacrylate, homomenthyl salicylate, parabens (in particular propyl and butyl paraben), methylisothiazolinone, chloromethylisothiazolinone and DMDM hydantoin, polyethylene glycol ethers or polyethylene glycol esters, and is free of mineral oils, silicone oils and mineral waxes.

14. Emulsion, use or process according to any of the preceding claims, **characterized in that** the preparation comprises one or more compounds selected from the group of compounds alpha-lipoic acid, folic acid, phytoene, D-biotin, coenzyme Q10, alpha-glucosylrutin, carnitine, carnosine, natural and/or synthetic isoflavonoids, flavonoids, creatine, creatinine, taurine, β-alanine, panthenol, magnolol, honokiol, tocopherol, tocopheryl acetate, dihydroxyacetone; polydocanol, Thiamidol, glycerylglucose, hyaluronic acid and/or salts thereof and/or licochalcone A.

## Revendications

1. Émulsion cosmétique éthanol-dans-glycérol, constituée par
a) de l'éthanol
b) une phase de glycérol
c) un émulsifiant
d) le cas échéant d'autres adjuvants actifs et additifs cosmétiques, qui se trouvent sous forme dissoute dans l'éthanol ou dans la phase de glycérol, **caractérisée en ce que** la teneur en eau de la préparation est inférieure à 5% en poids, par rapport au poids total de l'émulsion, et **caractérisée en ce qu'**un ou plusieurs poly(esters de glycéryle) ou de la lécithine de soja sont utilisés comme émulsifiant.

2. Utilisation d'une émulsion éthanol-dans-glycérol constituée par
a) de l'éthanol
b) une phase de glycérol
c) un émulsifiant, **caractérisée en ce qu'**un ou plusieurs poly(esters de glycéryle) ou de la lécithine de soja sont utilisés comme émulsifiant,
d) le cas échéant d'autres adjuvants actifs et additifs cosmétiques, qui se trouvent sous forme dissoute dans l'éthanol ou dans la phase de glycérol, en tant que cosmétique, **caractérisée en ce que** la teneur en eau de la préparation est inférieure à 5% en poids, par rapport au poids total de l'émulsion.

3. Procédé de préparation d'une émulsion éthanol-dans-glycérol, **caractérisé en ce que**, dans une première étape, l'émulsifiant est dissous dans de l'éthanol, dans une deuxième étape, la phase de glycérol est ajoutée sous agitation au mélange éthanol/émulsifiant et ensuite on homogénéise, **caractérisé en ce que** la teneur en eau de la préparation est inférieure à 5% en poids, par rapport au poids total de l'émulsion et **caractérisée en ce qu'**un ou plusieurs poly(esters de glycéryle) ou de la lécithine de soja sont utilisés comme émulsifiant.

4. Émulsion selon la revendication 1, utilisation selon la revendication 2 ou procédé selon la revendication 3, caractérisé(e) en ce que la teneur en éthanol de la préparation est de 5 à 25% en poids, par rapport au poids total de l'émulsion.

5. Émulsion, utilisation ou procédé selon l'une quelconque des revendications précédentes, **caractérisé en ce que** la phase de glycérine contient une ou plusieurs huiles choisies dans le groupe des composés comprenant le 2-cyano-3,3-diphénylacrylate d'éthylhexyle (INCI : Octocrylene), le 2-hydroxybenzoate d'éthyl-2-hexyle (INCI : Ethylhexyl Salicylate) et 2-hydroxybenzoate de 3,3,5-triméthylcyclohexyle (INCI : Homosalate).

6. Émulsion, utilisation ou procédé selon l'une quelconque des revendications précédentes, **caractérisé en ce que** la phase de glycérine contient du 2-hydroxybenzoate de 2-éthylhexyle (INCI : Ethylhexyl Salicylate) en une concentration de 1,0 à 7,5 % en poids par rapport au poids total de la préparation.

7. Émulsion, utilisation ou procédé selon l'une des revendications précédentes, caractérisé(e) en ce que la préparation contient du glycérol en une concentration de 60 à 90% en poids par rapport au poids total de la préparation.

8. Émulsion, utilisation ou procédé selon l'une quelconque des revendications précédentes, caractérisé(e) en ce que la préparation contient un ou plusieurs filtres UV choisis dans le groupe des composés 4-(tert-butyl)-4'-méthoxydibenzoylméthane, 2-[4-(diéthylamino)-2-hydroxybenzoyl]benzoate d'hexyle (INCI : diéthylamino hydroxybenzoyl hexyl benzoate), 2,4,6-tris-[anilino-(p-carbo-2'-ethyl-1'-hexyloxy)]-1,3,5-triazine (INCI : Ethylhexyl Triazone), la 2,4-bis-{[4-(2-éthyl-hexyloxy)-2-hydroxy]-phényl}-6-(4-méthoxyphényl)-1,3,5-triazine (INCI : Bis- Ethylhexyloxyphenol methoxyphenyl Triazine).

9. Émulsion, utilisation ou procédé selon l'une des revendications précédentes, caractérisé(e) en ce qu'on utilise, comme émulsifiant, un ester d'acide gras de polyglycéryle-10.

10. Émulsion, utilisation ou procédé selon l'une des revendications précédentes, caractérisé(e) en ce qu'on utilise, comme émulsifiant, un ester d'acide gras de polyglycéryle-10 en une concentration de 0,1 à 1,0% en poids, par rapport au poids total de la préparation.

11. Émulsion, utilisation ou procédé selon l'une des revendications précédentes, caractérisé(e) en ce qu'on utilise comme émulsifiant du stéarate de polyglycéryle-10 (INCI : Polyglyceryl-10 Stearate) et/ou de l'oléate de polyglycéryle-10 (INCI : Polyglyceryl-10 Oleate).

12. Émulsion, utilisation ou procédé selon l'une des revendications précédentes, caractérisé(e) en ce qu'on utilise, comme émulsifiant, de la lécithine de soja en une concentration de 0,1 à 1,0% en poids, par rapport au poids total de la préparation.

13. Émulsion, utilisation ou procédé selon l'une des revendications précédentes, caractérisé(e) en ce que la préparation est exempte de 3-(4-méthylbenzylidène)-camphre, de 2-hydroxy-4-méthoxybenzophénone (INCI : oxybenzone), de 4-méthoxycinnamate de 2-éthylhexyle (INCI : octyl méthoxycinnamate), de 2-cyano-3,3-diphénylacrylate de 2-éthylhexyle, de salicylate d'homomenthyle, de parabènes (en particulier de propylparabène et de butylparabène), de méthylisothiazolinone, de chlorométhylisothiazolinone et de DMDM-hydantoïne, d'éthers de polyéthylène glycol ou d'esters de polyéthylène glycol, et est en outre exempte d'huiles minérales, d'huiles de silicone et de cires minérales

14. Émulsion, utilisation ou procédé selon l'une des revendications précédentes, caractérisé(e) en ce que la préparation contient un ou plusieurs composés choisis dans le groupe des composés acide alpha-liponique, acide folique, phytoène, D-biotine, coenzyme Q10, alpha-glucosylrutine, carnitine, carnosine, isoflavonoïdes naturels et/ou synthétiques, flavonoïdes, créatine, créatinine, taurine, β-alanine, panthénol, magnolol, honokiol, tocophérol, acétate de tocophéryle, dihydroxyacétone ; polydocanol, thiamidol, glycérylglucose, acide hyaluronique et/ou ses sels et/ou licochalcone A.
